# EUROPEAN PATENT APPLICATION

(11) **EP 3 964 207 A1**
(43) Date of publication of application: **09.03.2022**
(21) Application number: 20194171.3
(22) Date of filing: 02.09.2020
(51) Int. Cl.: A61K 31/25, A61P 31/12

(54) **ANTIVIRAL PHARMACEUTICAL COMPOSITION FOR TOPICAL ADMINISTRATION**

(71) Applicant: Christian-Albrechts-Universität zu Kiel, 24118 Kiel (DE)
(72) Inventor: SCHADE, Dennis, 24114 Kiel (DE); SCHERLIEß, Regina, 24106 Kiel (DE); CLEMENT, Bernd, 24161 Kiel (DE)
(74) Representative: Uexküll & Stolberg

(57) **Abstract**

The present invention relates to a topical pharmaceutical composition comprising a serine protease inhibitor for use in the treatment or prevention of viral infections. The invention is further directed to serine protease inhibitors and a lozenge and a dry nasal preparation comprising the serine protease inhibitor.

## Description

The present invention relates to a topical pharmaceutical composition for use in the treatment or prevention of viral infections.

Viral infections pose a major threat to human health. Virus outbreaks, such as local outbreaks, epidemics or pandemics, can also have for-reaching consequences for societies and economies, as the pandemic of coronavirus disease 2019 (COVID-19) caused by severe acute respiratory syndrome coronavirus 2 (SARS-CoV-2) has shown.

In such an outbreak event, on the one hand there is a great need for means of treating infected patients. On the other hand, there is also a great need for measures that can be effective in containing the outbreak by preventing virus transmission events.

There are several different approaches for the treatment of viral infections. Since viruses depend on replication inside host cells, one treatment strategy is to inhibit viral entry, e.g. by interfering with binding of a virion to a host cell or fusion of viral and cellular membranes. A specific treatment method targets cellular proteases which, by processing viral surface proteins, facilitate viral entry. Inhibition of such cellular proteases is intended to result in a reduced viral entry. For example, EP 3 536 342 A1 describes a pharmaceutical composition comprising an inhibitor for cellular transmembrane serine proteases for use in treating or preventing influenza virus infection.

Camostat mesylate and nafamostat mesylate are protease inhibitors which have been approved for the treatment of chronic pancreatitis but have also been described to have antiviral activity. For example, camostat was described to inhibit the cellular "transmembrane protease, serine 2" (TMPRSS2) and to display antiviral activity in a pathogenic animal model for SARS-CoV infection, indicating that serine protease inhibitors are suitable for treatment of SARS and potentially Middle East respiratory virus (MERS) causes by MERS-CoV (Zhou et al., Antiviral Research 116 (2015) 76-84). In the course of the COVID-19 pandemic, important commonalities between SARS-CoV-2 and SARS-CoV infections were shown, SARS-CoV-2 cell entry was demonstrated to depend on TMPRSS2, and camostat mesylate and nafamostat mesylate were demonstrated to reduce SARS-CoV-2 infections of lung cells (Hoffmann et al., Cell (2020), 181, 271-280 and Hoffmann et al., Antimicrob. Agents Chemother. 64:e00754-20) .

Serine protease inhibitors have been developed for the administration via the enteral route and are typically provided as solid dosage forms such as tablets, also due to the practical advantages for medical staff and patients. For the approved indications of camostat mesylate, i.e. for treating chronic pancreatitis and postoperative reflux esophagitis, the typical daily dose is 600 mg or 300 mg, respectively. In clinical studies concerning the treatment of chronic pancreatitis, camostat mesylate was reported to cause adverse effects in 1.8% of the treated individuals with the most common effects being rash, pruritus, nausea, abdominal discomfort, and abdominal fullness.

In the case of treating SARS-CoV-2 infections with camostat mesylate it was assumed that the daily intake of 600 mg active substance in the form of tablets could have positive effect on a SARS-CoV-2 infection in a patient (Uno, Intern. Emerg. Med. (2020) Apr 29: 1-2).

In addition to the active ingredient camostat mesylate, the pharmaceutical composition of the commercial product (FOIPAN^{®} by Ono Pharmaceutical Co., Ltd.) comprises excipients that are commonly used in oral tablets, namely hydroxypropyl cellulose, carmellose calcium, magnesium stearate, polyoxyethylene (105), polyoxyethylene(5)glycol, and lactose hydrate.

In the beginning of August 2020, eight different clinical trials related to the treatment of SARS-CoV-2 infections with camostat mesylate were internationally registered (www.clinicaltrials.gov). In all these studies, camostat mesylate is or is planned to be administered via the enteral route at a total daily dose of at least 600 mg.

Thus, the present invention is based on the objective of providing a pharmaceutical composition for use in a method for the effective treatment or prevention of viral infections, in particular SARS-CoV2 infections, which composition has a rapid onset of action, a simple and convenient mode of administration and minimum side effects.

These objects are achieved by the pharmaceutical composition according to claims 1 to 12. The invention also relates to the serine protease inhibitors according to claim 13, the lozenge according to claim 16, and the dry or liquid nasal preparation according to claim 15.

In a first aspect, the present invention relates to a pharmaceutical composition comprising a serine protease inhibitor for use in a method for the treatment or prevention of viral infections, wherein the pharmaceutical composition is administered topically.

It has surprisingly been found that the topical administration of a pharmaceutical composition comprising a serine protease inhibitor is suitable for use in a method for the treatment or prevention of viral infections and is associated with several advantages over the commonly used enteral administration.

Firstly, the topical administration route allows treatment and prevention of viral infection by using surprisingly low amounts of serine protease inhibitor. Compared to the compositions of the prior art, which are administered via the enteric route, much less serine protease inhibitor is sufficient to achieve the desired therapeutic effect.

Moreover, the topical administration of serine protease inhibitors considerably reduces the risk of adverse side effects affecting the person's physical condition. This reduction of the risk of side effects is believed to be not only caused by dose-dependent effects, i.e. by the low amounts of serine protease inhibitors that can be used by topical administration, but also by the reduced extent to which the serine protease inhibitor is distributed in the treated person's body.

It was also found that the topical administration is accompanied with a surprisingly rapid onset of action when treating viral infections affecting topical tissues. This is believed to be related to the fact that the absorption of the serine protease inhibitor in the gastrointestinal tract and the transport to the infected tissue are no longer necessary in case of a topical administration.

According to the present invention, the term "topically administered" relates to an administration method involving that the pharmaceutical composition is applied directly onto the topical tissue at which the inhibitory effect of the serine protease inhibitor is desired or that the pharmaceutical composition is brought into close proximity of this topical tissue. Hence, the serine protease inhibitor causes a local inhibitory effect in the topical tissue which has been exposed to the composition and in its local vicinity.

The pharmaceutical composition according to the present invention is suitable for the treatment and prevention of viral infections. Treating a viral infection may involve impairing the viral replication cycle, reducing the viral load in the infected person and/or reducing the symptoms caused by the viral infection or by the infected person's immune response. Preventing a viral infection may involve reducing a person's infectiousness, i.e. the likelihood for an infected person to infect another individual, and/or a person's susceptibility for infection, i.e. the likelihood for a person to be infected by another infected individual.

Viral infections, which may be treated or prevented using the pharmaceutical composition of the present invention, may be viral infections affecting a topical tissue, i.e. particularly a mucosa. In a preferred embodiment the pharmaceuticals composition is intended for use in a method for the treatment or prevention of viral infections affecting at least part of the respiratory tract and/or the eyes and in particular the upper respiratory tract.

According to the invention, the respiratory tract comprises the upper and the lower respiratory tract. When reference is made to the upper respiratory tract it is referred to at least part of one or more of the nose, the nasal cavity, the oral cavity, the paranasal sinuses, the larynx and the pharynx, such as at least part of one or more of the nasopharynx, the oropharynx and the laryngopharynx. When reference is made to the lower respiratory tract it is referred to at least part of one or more of the trachea, the conducting airways, the bronchi, the bronchioles, and the alveoli.

In a preferred embodiment, the pharmaceutical composition is suitable for use in a method for the treatment or prevention of a viral infection that is caused by a virus infecting the respiratory tract, in particular a virus selected from the group consisting of coronaviruses, influenza viruses, parainfluenza viruses, adenoviruses, rhinoviruses, respiratory syncytial viruses, more preferably by coronaviruses and/or influenza viruses, particularly preferably by SARS-CoV-2, MERS-CoV and related coronaviruses, and most preferably by SARS-CoV-2.

The pharmaceutical composition may be used for a method of treatment or prevention of a viral infection before (presymptomatic) or after the onset of symptoms that are associated with the viral infection and may also be used for a method of treatment or prevention of asymptomatically infected individuals.

In the case of treating or preventing SARS-CoV-2 infections the method for the treatment or prevention may e.g. involve the topical administration before infection, before the onset of symptoms associated with the infection, in the first 17 days after infection or in the first 10 days after the onset of symptoms.

According to the present invention, the pharmaceutical compositions may be topically administered to individuals, who have an increased risk of being infected with a viral infection, e.g. because they are particularly susceptible to the viral infection, or are at particular risk from the effects of the viral infection, e.g. due to pre-existing health conditions or high age.

The pharmaceutical composition may also be topically administered to individuals spending time in areas where infections are expected to be more likely to occur, e.g. medical settings such as hospitals or medical practices, in households where infected people live, in schools, in mass gatherings, in mass events, or in public transportation.

The high concentration of serine protease inhibitor at the tissue of interest combined with a lower probability of side effects caused by the topically administered pharmaceutical composition compared to enterally administered pharmaceutical compositions of the prior art make the pharmaceutical composition of the present invention particularly suitable for a method for treatment or prevention of viral infections in asymptomatic, presymptomatic or mildly symptomatic individuals, as these individuals typically shall be particularly protected from side effects when taking into account a risk-benefit assessment.

The method of treatment or prevention of viral infections may involve the treatment or prevention of a viral infection in a human body or in an animal body, preferably in a human body or in a non-human mammalian body, particularly preferably in a human body.

The pharmaceutical composition may be topically administered to a mucosa of the upper respiratory tract, the lower respiratory tract or the eye. In a preferred embodiment the pharmaceutical composition is topically administered to a mucosa, preferably to a mucosa of the upper respiratory tract or the eye, particularly preferably to a mucosa of the upper respiratory tract.

The term "mucosa" refers to a moist topical tissue covering inner surfaces of the body and typically comprises one or several layers of epithelial cells, a loose collageneous connective tissue (lamina propria), and optionally a mucosal muscle layer.

The topical administration to a mucosa in the upper respiratory tract preferably comprises the administration to a mucosa of at least part of one or more of the nose, the nasal cavity, the oral cavity, the paranasal sinuses, the larynx and the pharynx, such as at least part of one or more of the nasopharynx, the oropharynx and the laryngopharynx.

It is particularly preferred that the pharmaceutical composition is topically administered to a mucosa for local therapy.

In an embodiment of the invention, the pharmaceutical composition is administered in the form of a lozenge, a mucoadhesive buccal preparation, a medicated chewing gum, a nasal preparation, an inhalation product, an ophthalmic ointment or eye drops, preferably as a lozenge or as a nasal preparation.

Nasal preparations may be liquid or dry nasal preparations. Preferred liquid nasal preparations include sprays, drops, creams, ointments, and gels. Preferred dry nasal preparations include powders and microspheres. The administration of such liquid and dry preparations preferably includes the use of liquid or dry powder unit dose or multi dose nasal devices.

It is preferred to administer nasal preparations in the form of dry nasal preparations. Particularly preferred are nasal preparations in the form of dry nasal powders.

Preferred inhalation products are liquid or dry powder preparations which are administered with respective inhalers, such as pressurized and non-pressurized metered dose inhalers, dry powder inhalers, soft mist inhalers, and nebulizers.

Typically, the daily dose required for treating or preventing a viral infection using a pharmaceutical composition according to the present invention is lower than the dose required using pharmaceutical compositions of the prior art.

In one embodiment, the pharmaceutical composition is administered with a maximum daily dose of serine protease inhibitor of 400 mg or less, preferably 100 mg or less, and particularly preferably 10 mg or less.

In a preferred embodiment, in which the pharmaceutical composition is administered in the form of a lozenge, the pharmaceutical composition is administered with a maximum daily dose of serine protease inhibitor of 300 mg or less, preferably 250 mg or less, and particularly preferably 200 mg or less.

It may be preferred to administer the pharmaceutical composition in the form of a lozenge with a maximum daily dose of serine protease inhibitor in the range of 5 to 300 mg, preferably 10 to 250 mg, in particular 20 to 200 mg.

In another embodiment, in which the pharmaceutical composition is administered in the form of a nasal preparation, the pharmaceutical is administered with a maximum daily dose of serine protease inhibitor of 100 mg or less, preferably 30 mg or less, and particularly preferably 1 mg or less.

It may be preferred to administer the pharmaceutical composition in the form of a nasal preparation with a maximum daily dose of serine protease inhibitor in the range of 0.01 to 100 mg, preferably 0.05 to 30 mg, in particular 0.1 to 1 mg.

Typically, the pharmaceutical composition of the present invention also comprises a lower amount of serine protease inhibitor in a single dosage form than pharmaceutical compositions of the prior art.

In one embodiment the pharmaceutical composition has an absolute dose of serine protease inhibitor per single dosage form of 50 mg or less, preferably 20 mg or less, and particularly preferably 2 mg or less.

In a preferred embodiment the pharmaceutical composition is administered in the form of a lozenge having an absolute dose of serine protease inhibitor per single dosage form of 20 mg or less, preferably 0.05 to 10 mg, and particularly preferably 0.2 to 5 mg.

In another preferred embodiment the pharmaceutical composition is administered in the form of a nasal preparation, such as a dry or liquid nasal preparation, having an absolute dose of serine protease inhibitor per single dosage form of 1 mg or less, preferably 1 to 500 µg, and particularly preferably 5 to 200 µg, such as 5 to 50 µg.

The topical administration of a single dosage form of the pharmaceutical preparation according to the present invention preferably results in a plasma concentration of serine protease inhibitor and its active metabolites combined which is too low to result in an effective inhibition of the targeted serine protease.

For example, the administration of a single dosage form of the pharmaceutical composition to a fasted individual may provide a peak plasma concentration (Cₘₐₓ) of the serine protease inhibitor and its active metabolites combined that is lower than their effective concentration at 10%, preferably 5%, particularly preferably 3% inhibition of the targeted serine protease.

In a preferred embodiment the administration of a single dosage form of the pharmaceutical composition to a fasted individual provides a peak plasma concentration (Cₘₐₓ) of the serine protease inhibitor and its active metabolites combined of 0.3 µM or less, preferably 0.1 µM or less, particularly preferably 0.03 µM or less, and most preferably 0.01 µM or less. For example, administration of a single dosage form may provide a peak plasma concentration (Cₘₐₓ) of the serine protease inhibitor in the range of 0.01 nM to 0.3 µM. In a particularly preferred embodiment the serine protease inhibitor that is topically administered and results in the above peak plasma concentrations is selected from the group consisting of camostat, nafamostat, derivatives thereof and serine protease inhibitors according to the second aspect of the invention. The peak plasma concentration can be measured by methods well known in the art, including liquid chromatography mass spectrometry.

The pharmaceutical composition comprises a serine protease inhibitor targeting one or more serine proteases. In a preferred embodiment the serine protease that is inhibited by the serine protease inhibitor is a transmembrane protease, preferably a type II transmembrane protease. According to the present invention, a transmembrane protease is a protease comprising at least one peptide sequence spanning the cell membrane. A type II membrane protease comprises a single membrane spanning peptide sequence and has a C-terminus that is located at the extracellular side.

It is particularly preferred that the serine protease inhibited by the serine protease inhibitor is TMPRSS2.

In another embodiment, the serine protease inhibited by the serine protease inhibitor is a viral protease.

According to the present invention, a serine protease inhibitor is suitable for inhibiting a serine protease, which may involve preventing or decreasing the rate of a proteolytic reaction of the serine protease.

The serine protease inhibitor may be a covalent or noncovalent inhibitor and may inhibit the serine protease reversibly or irreversibly. Preferably, the serine protease inhibitor is a covalent inhibitor and/or inhibits the serine protease reversibly.

In the case of camostat binding to TMPRSS2, the inhibitory effect may for instance be caused by an acylation of a serine residue 441 of TMPRSS2 in a reaction according to the following reaction scheme:

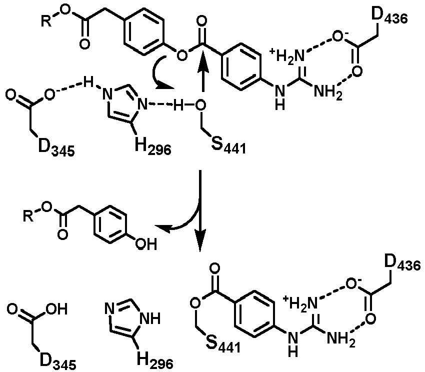

In a preferred embodiment, the serine protease inhibitor in the pharmaceutical composition according to the invention is a compound of Formula (I) or (II) 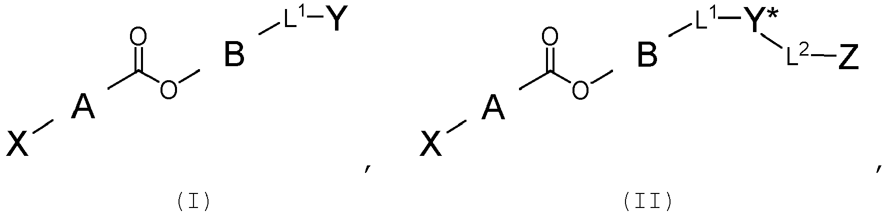
wherein A and B are independently selected from aryl and heteroaryl,
X is selected from the group consisting of 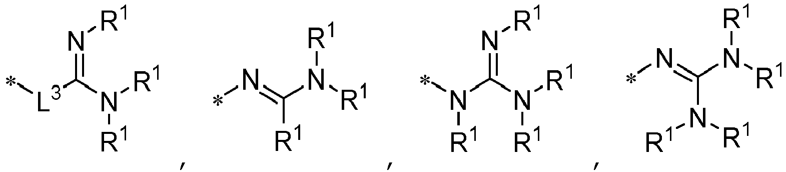 and 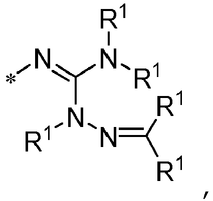 wherein
   L³ is an optional linking group selected from the group consisting of alkyl, heteroalkyl, alkenyl, heteroalkenyl, alkynyl, alkylaryl, heteroalkylaryl, heteroalkynyl, cycloalkyl, heterocycloalkyl, aryl, and heteroaryl, and preferably is a linear or branched alkyl having up to 4 carbon atoms, more preferably 1 or 2 carbon atoms,
   R¹ is independently selected from the group consisting of H, alkyl, heteroalkyl, alkenyl, heteroalkenyl, alkynyl, heteroalkynyl, cycloalkyl, heterocycloalkyl, aryl, heteroaryl, alkylaryl, heteroalkylaryl, and preferably selected from H, alkyl and heteroalkyl, wherein said alkyl and heteroalkyl have up to 3 carbon atoms and are optionally joined to another R¹ to form a heterocycle, and more preferably R¹ is H,
Y is an acidic functional group, preferably selected from the group consisting of carboxylic acid, sulfonic acid, phosphonic acid, phosphinic acid, sulfonamides, and hydroxamic acid, and esters and inverted esters thereof,
Y* is a hydrolysable derivative from an acidic functional group, preferably selected from the group consisting of ester, inverted ester, amide, carbamate, and anhydride,
Z is an N-containing group and is preferably selected from the group consisting of N-containing heteroalkyl and alkylamide moieties, each having preferably up to 6 carbon atoms, and N-containing monocyclic and bicyclic heterocyclyl and heteroaryl,
L¹ and L² are optional linking groups which are present or absent and which are independently selected from the group consisting of alkyl, heteroalkyl, alkenyl, heteroalkenyl, alkynyl, alkylaryl, heteroalkylaryl, heteroalkynyl, cycloalkyl, heterocycloalkyl, aryl, and heteroaryl, and preferably are linear or branched alkyl having up to 4 carbon atoms,
or a pharmaceutically acceptable salt thereof.

Accordingly, X may be a cyclic or linear amidine, guanidine or amidinohydrazone.

For example, if X is 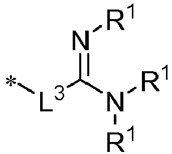 it may be 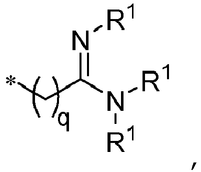 wherein q is an integer from 0 to 2.

In a preferred embodiment, X is guanidine.

Z may, for example, be selected from the group consisting of 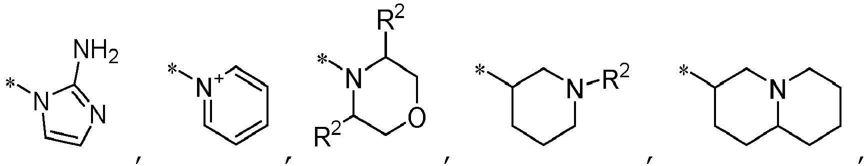 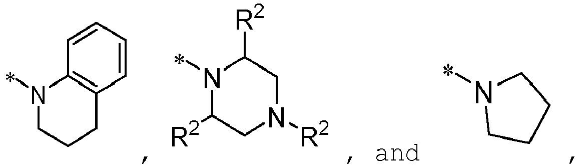 wherein R² is independently selected from the group consisting of H, alkyl, heteroalkyl, alkenyl, heteroalkenyl, alkynyl, heteroalkynyl, cycloalkyl, heterocycloalkyl, aryl, heteroaryl, alkylaryl, heteroalkylaryl, and preferably selected from the group consisting of H, alkyl, heteroalkyl, cycloalkyl and heterocycloalkyl, wherein said alkyl, heteroalkyl, cycloalkyl or heterocycloalkyl can have up to 6 carbon atoms.

In one embodiment, Z is bound to the compound of Formula (II) via an N atom. In another embodiment, Z is bound to the compound of Formula (II) via a carbon atom.

More preferably, Z is selected from N-alkylamino and quaternary ammonium moieties and in particular from 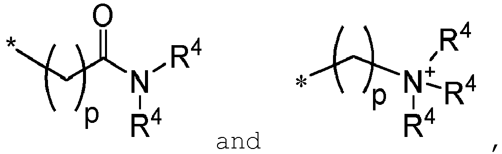
wherein p is an integer from 0 to 4, and
R⁴ is independently selected from the group consisting of H, alkyl, heteroalkyl, alkenyl, heteroalkenyl, alkynyl, heteroalkynyl, cycloalkyl, heterocycloalkyl, aryl, heteroaryl, alkylaryl, heteroalkylaryl, and preferably selected from H, alkyl and heteroalkyl, wherein said alkyl and heteroalkyl may have up to 3 carbon atoms, and is optionally joined to L² or to another R⁴ to form a heterocycle, and more preferably R⁴ is methyl or H, and
particularly preferably Z is selected from the group consisting of 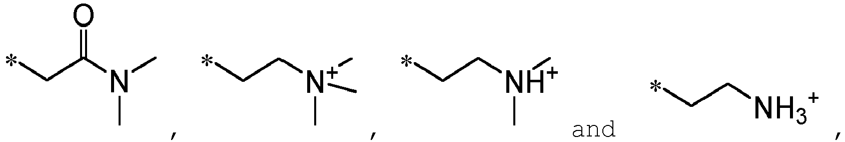 with L² being absent.

It is further preferred that Z comprises a group which is permanently positively charged or positively or negatively charged at a pH value in the range of pH 5.5 to pH 7.5, particularly preferably at a pH value in the range of pH 6 to pH 7.

Moreover, it is preferred that A and B are independently selected from monocyclic, bicyclic or tricyclic aryl or heteroaryl and are preferably phenyl or naphthyl, particularly preferably phenyl.

It is preferred that Y is selected from the group consisting of 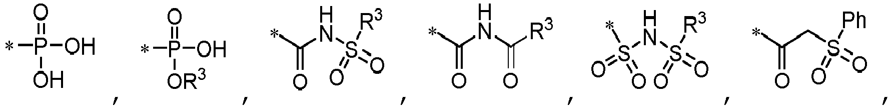 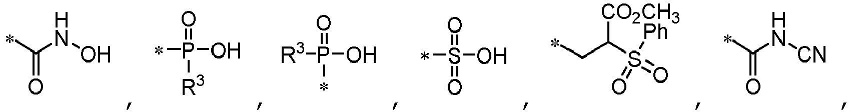 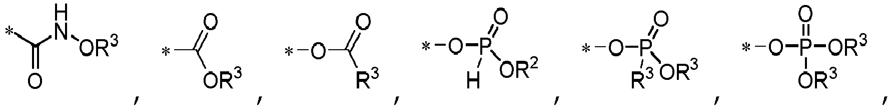 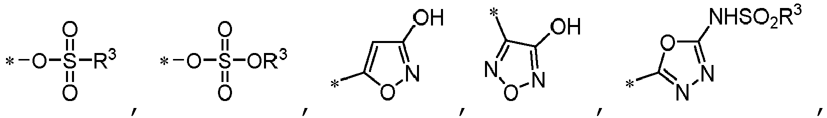 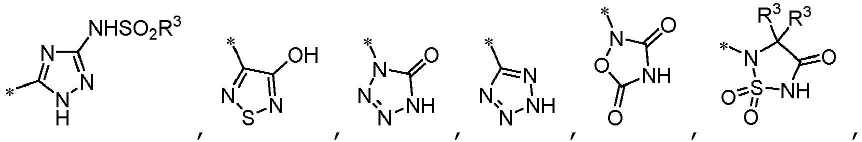 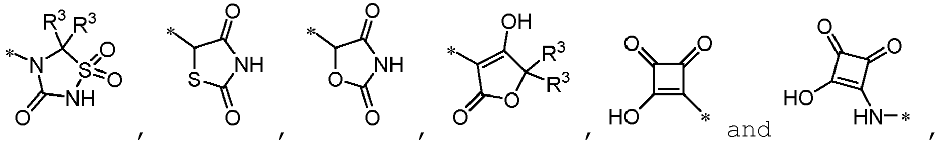
wherein Ph is phenyl,
R³ is independently selected from the group consisting of H, alkyl, heteroalkyl, alkenyl, heteroalkenyl, alkynyl, heteroalkynyl, cycloalkyl, heterocycloalkyl, aryl, heteroaryl, alkylaryl, heteroalkylaryl, and preferably selected from the group consisting of H, alkyl, heteroalkyl, cycloalkyl and heterocycloalkyl, wherein said alkyl, heteroalkyl, cycloalkyl or heterocycloalkyl can have up to 6 carbon atoms.

It is also preferred that Y* is an ester group or an inverted ester group, preferably a carboxylic acid ester, sulphonic acid ester, phosphonic acid ester, phosphoric acid ester or phosphonic acid diester, and more preferably a carboxylic acid ester.

In another embodiment, the serine protease inhibitor is a compound of Formula (I), wherein A and B are phenyl, L¹ is a linear or branched alkyl or heteroalkyl having up to 4 carbon atoms, and Y is carboxylic acid.

According to one embodiment, the serine protease inhibitor of Formula (I) is not camostat. According to another embodiment, the serine protease inhibitor is not nafamostat.

In a particularly preferred embodiment the serine protease inhibitor in the pharmaceutical composition according to the invention is a compound of Formula (I) or (II), wherein

A and B are independently selected from monocyclic, bicyclic or tricyclic aryl or heteroaryl and are preferably phenyl or naphthyl, particularly preferably phenyl,
Y is selected from the group consisting of 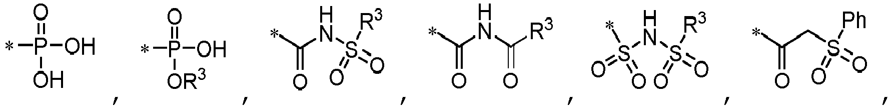 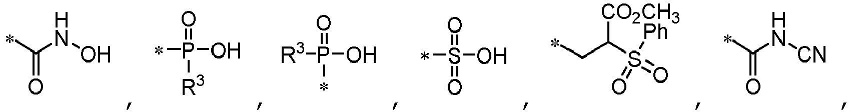 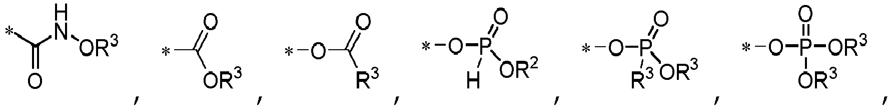 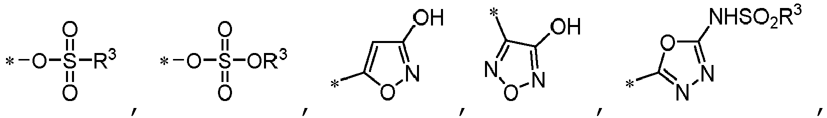 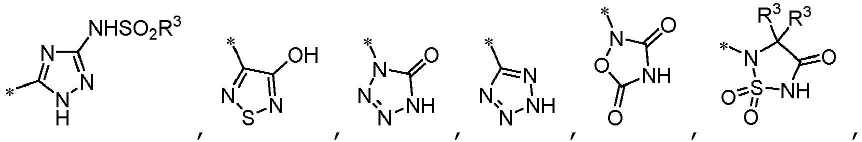 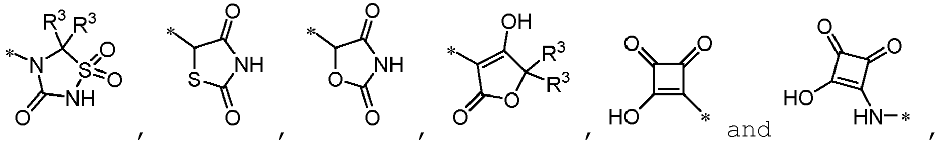
wherein Ph is phenyl,
R³ is independently selected from the group consisting of H, alkyl, heteroalkyl, alkenyl, heteroalkenyl, alkynyl, heteroalkynyl, cycloalkyl, heterocycloalkyl, aryl, heteroaryl, alkylaryl, heteroalkylaryl, and preferably selected from the group consisting of H, alkyl, heteroalkyl, cycloalkyl and heterocycloalkyl, wherein said alkyl, heteroalkyl, cycloalkyl or heterocycloalkyl can have up to 6 carbon atoms,
Y* is an ester group or an inverted ester group, preferably a carboxylic acid ester, sulphonic acid ester, phosphonic acid ester, phosphoric acid ester or phosphonic acid diester, and more preferably a carboxylic acid ester,
Z is selected from N-alkylamino and quaternary ammonium moieties and preferably from 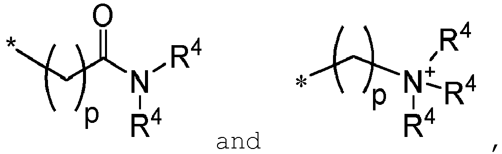
wherein p is an integer from 0 to 4, preferably 1 or 2, and
R⁴ is independently selected from the group consisting of H, alkyl, heteroalkyl, alkenyl, heteroalkenyl, alkynyl, heteroalkynyl, cycloalkyl, heterocycloalkyl, aryl, heteroaryl, alkylaryl, heteroalkylaryl, and preferably selected from H, alkyl and heteroalkyl, wherein said alkyl and heteroalkyl may have up to 3 carbon atoms, and is optionally joined to L² or to another R⁴ to form a heterocycle, and more preferably R⁴ is methyl or H, and
more preferably Z is selected from the group consisting of 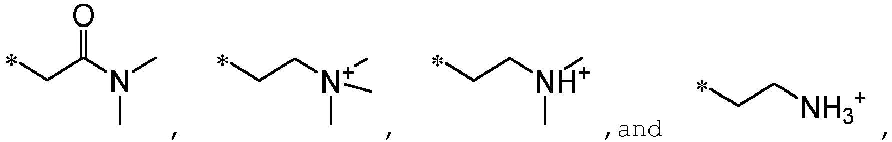 with L² being absent,
or a pharmaceutically acceptable salt thereof.

In a preferred embodiment, R¹, R², R³ and/or R⁴ comprise a moiety independently selected from the group consisting of carboxylic acids, peroxycarboxylic acids, thiocarboxylic acids, sulfonic acids, sulfenic acids, sulfinic acids, sulfoxides, carboxylic acid anhydrides, carboxylic acid esters, sulfonic acid esters, carboxylic acid amides, sulfonic acid amides, carboxylic acid hydrazides, carboxylic acid halides, sulfonic acid halides, nitriles, aldehydes, ketones, thioaldehydes, thioketones, oximes, N-oxides, hydrazones, alcohols, phenols, thiols, amines, amidines, guanidines, imines, hydrazines, ethers, esters, thioethers, nitro, nitroso, azo, diazo, isocyanides, isoscyanates, thiocyanates, isothiocyanates, hydroperoxides and/or peroxides.

In a particularly preferred embodiment the serine protease inhibitor in the pharmaceutical composition according to the invention is a compound having a structure of Formula (III) 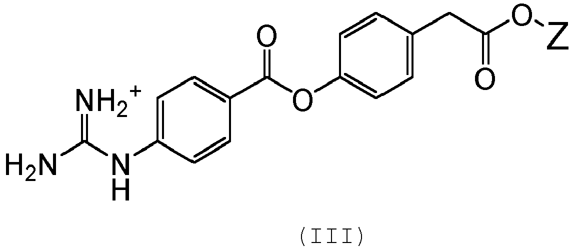 with Z being selected from the group consisting of H, 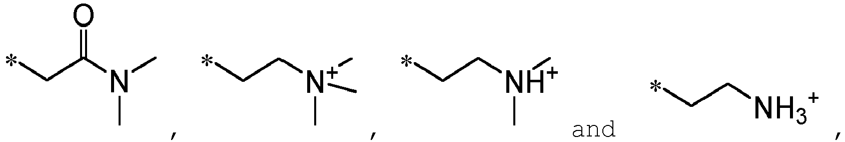 or a pharmaceutically acceptable salt thereof, and preferably is camostat mesylate.

In a preferred embodiment, the serine protease inhibitor in the pharmaceutical composition is a compound of Formula (III) and Z is H. Thus, the compound is preferably present as a free carboxylic acid. However, under physiological conditions the free acid is deprotonated resulting in a negatively charged carboxylate.

It was surprisingly found that pharmaceutical compositions comprising an serine protease inhibitor having a structure of Formula (III) wherein Z is H is suitable for use in a method for the treatment or prevention of viral infections despite the compound's low solubility that is associated with the fact that the compound is present as a zwitterion at physiological pH.

In another embodiment of the first aspect of the invention the serine protease inhibitor in the pharmaceutical composition according to the invention is a compound having a structure of Formula (IV) 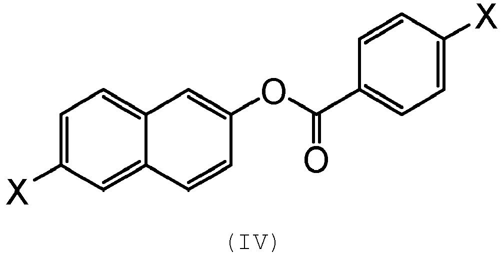 wherein X is independently selected from the group consisting of 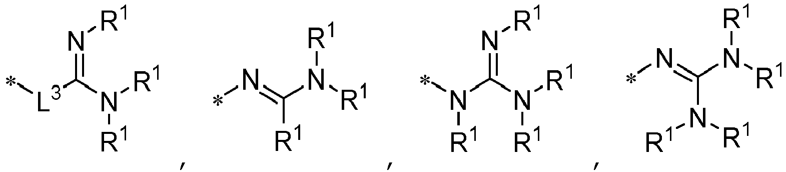 and 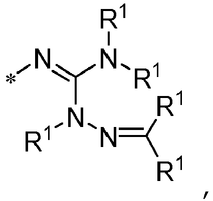 with R¹ being independently selected from the group consisting of H, alkyl, heteroalkyl, alkenyl, heteroalkenyl, alkynyl, heteroalkynyl, cycloalkyl, heterocycloalkyl, aryl, heteroaryl, alkylaryl, heteroalkylaryl, and preferably selected from H, alkyl and heteroalkyl, wherein said alkyl and heteroalkyl have up to 3 carbon atoms and are optionally joined to another R¹ to form a heterocycle, and more preferably R¹ is H,

Accordingly, X may be a cyclic or linear amidine, guanidine or aminidinohydrazone.

For example, if X is 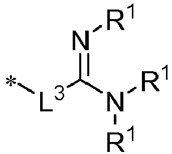 it may be 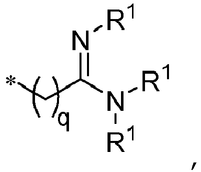 wherein q is an integer from 0 to 2.

In a particularly preferred embodiment the protease inhibitor may be nafamostat or a pharmaceutically acceptable salt thereof, particularly nafamostat mesylate.

It is preferred that after topical administration the serine protease inhibitor or an active metabolite thereof is present as a zwitterion at the target site. This was surprisingly found to ensure a high on-target exposition on topical tissues, such as mucosal membranes, while avoiding a systemic availability. In contrast to this, in pharmaceutical compositions for enteral application a zwitterionic state of the active substance is usually avoided due to the associated low solubility of the zwitterionic active substance.

In a preferred embodiment, the serine protease inhibitor is present as a zwitterion at a pH value that is in the range of pH 5.5 to pH 7.5. If the pharmaceutical composition is a nasal preparation, it is particularly preferred that the serine protease inhibitor is present as a zwitterion at a pH value that is in the range of pH 5.5 to pH 6.5. If the pharmaceutical composition is a lozenge, a mucoadhesive buccal preparation or a medicated chewing gum, it is particularly preferred that the serine protease inhibitor is present as a zwitterion at a pH value that is in the range of pH 6 to pH 7. If the pharmaceutical composition is an inhalation product, it is particularly preferred that the serine protease inhibitor is present as a zwitterion at a pH value that is in the range of pH 7 to pH 7.5.

As used herein, "Alkyl" means an aliphatic hydrocarbon group which may be straight or branched and comprising about 1 to about 20 carbon atoms in the chain. Preferred alkyl groups contain about 1 to about 12 carbon atoms in the chain. More preferred alkyl groups contain about 1 to about 6 carbon atoms in the chain. Branched means that one or more lower alkyl groups such as methyl, ethyl or propyl, are attached to a linear alkyl chain. "Lower alkyl" means a group having about 1 to about 6 carbon atoms in the chain which may be straight or branched. The term "substituted alkyl" means that the alkyl group may be substituted by one or more substituents which may be the same or different, each substituent being independently selected from the group consisting of halo, alkyl, aryl, cycloalkyl, cyano, hydroxy, alkoxy, alkylthio, amino,-NH(alkyl), -NH(cycloalkyl), -N(alkyl)₂, carboxy and -C(O)O-alkyl. Non-limiting examples of suitable alkyl groups include methyl, ethyl, n-propyl, isopropyl and t-butyl.

"Heteroalkyl" means an alkyl moiety as defined above, having one or more carbon atoms, for example one, two or three carbon atoms, replaced with one or more heteroatoms, which may be the same or different, where the point of attachment to the remainder of the molecule is through a carbon atom of the heteroalkyl radical. Suitable such heteroatoms include O, S, S(O), S(O)2, and -NH-, -N(alkyl)-. Non-limiting examples include ethers, thioethers, amines, hydroxymethyl, 3-hydroxypropyl, 1,2-dihydroxyethyl, 2-methoxyethyl, 2-aminoethyl, 2-dimethylaminoethyl, and the like. Heteroalkyl chains may be unsubstituted or substituted with from 1 to 4 substituents. Preferred substituted heteroalkyl are mono-, di-, or tri-substituted. Heteroalkyl may be substituted with lower alkyl, haloalkyl, halo, hydroxy, aryloxy, heteroaryloxy, acyloxy, carboxy, monocyclic aryl, heteroaryl, cycloalkyl, heterocycloalkyl, spirocycle, amino, acylamino, amido, keto, thioketo, cyano, or any combination thereof.

"Alkenyl" means an aliphatic hydrocarbon group containing at least one carbon-carbon double bond and which may be straight or branched and comprising about 2 to about 20, preferably about 2 to about 15, carbon atoms in the chain. Preferred alkenyl groups have about 2 to about 12 carbon atoms in the chain; and more preferably about 2 to about 4 carbon atoms in the chain. Branched means that one or more lower alkyl groups such as methyl, ethyl or propyl, are attached to a linear alkenyl chain. Non-limiting examples of suitable alkenyl groups include ethenyl and propenyl. The term "substituted alkenyl" means that the alkenyl group may be substituted by one or more substituents which may be the same or different, each substituent being independently selected from the group consisting of alkyl, aryl and cycloalkyl.

"Heteroalkenyl" means a heteroalkyl as defined above having at least one double bond.

"Alkynyl" means an aliphatic hydrocarbon group containing at least one carbon-carbon triple bond and which may be straight or branched and comprising about 2 to about 20, preferably about 2 to about 15, carbon atoms in the chain. Preferred alkynyl groups have about 2 to about 12 carbon atoms in the chain; and more preferably about 2 to about 4 carbon atoms in the chain. Branched means that one or more lower alkyl groups such as methyl, ethyl or propyl, are attached to a linear alkynyl chain. Non-limiting examples of suitable alkynyl groups include ethynyl, propynyl, 2-butynyl and 3-methylbutynyl. The term "substituted alkynyl" means that the alkynyl group may be substituted by one or more substituents which may be the same or different, each substituent being independently selected from the group consisting of alkyl, aryl and cycloalkyl.

"Heteroalkynyl" means a heteroalkyl as defined above having at least one triple bond.

"Aryl" means an aromatic monocyclic or multicyclic ring system comprising about 6 to about 20 carbon atoms, preferably about 6 to about 14 carbon atoms, particularly preferably about 6 to about 10 carbon atoms. The aryl group can be optionally substituted with one or more "ring system substituents" which may be the same or different, and are as defined herein. Non-limiting examples of suitable aryl groups include phenyl and naphthyl.

"Heteroaryl" means an aromatic monocyclic or multicyclic ring system comprising about 5 to about 20 ring atoms, preferably about 5 to about 14 ring atoms, particularly preferably about 5 to about 10 ring atoms, in which one or more of the ring atoms is an element other than carbon, for example nitrogen, oxygen or sulfur, alone or in combination. Preferred heteroaryls contain about 5 to about 6 ring atoms. The "heteroaryl" can be optionally substituted by one or more "ring system substituents" which may be the same or different, and are as defined herein. The prefix aza, oxa or thia before the heteroaryl root name means that at least a nitrogen, oxygen or sulfur atom respectively, is present as a ring atom. A nitrogen atom of a heteroaryl can be optionally oxidized to the corresponding N-oxide. Non-limiting examples of suitable heteroaryls include pyridyl, pyrazinyl, furanyl, thienyl, pyrimidinyl, pyridone (including N-substituted pyridones), isoxazolyl, isothiazolyl, oxazolyl, thiazolyl, pyrazolyl, furazanyl, pyrrolyl, pyrazolyl, triazolyl, 1,2,4-thiadiazolyl, pyrazinyl, pyridazinyl, quinoxalinyl, phthalazinyl, oxindolyl, imidazo[1,2-a]pyridinyl, imidazo[2,1-b]thiazolyl, benzofurazanyl, indolyl, azaindolyl, benzimidazolyl, benzothienyl, quinolinyl, imidazolyl, thienopyridyl, quinazolinyl, thienopyrimidyl, pyrrolopyridyl, imidazopyridyl, isoquinolinyl, benzoazaindolyl, 1,2,4-triazinyl, benzothiazolyl and the like. The term "heteroaryl" also refers to partially saturated heteroaryl moieties such as, for example, tetrahydroisoquinolyl, tetrahydroquinolyl and the like.

"Alkylaryl" means an alkyl-aryl-group in which the alkyl and aryl are as previously described. Preferred alkylaryls comprise a lower alkyl group. Non-limiting example of a suitable alkylaryl group is tolyl. The bond to the parent moiety is through the aryl.

"Heteroalkylaryl" means a heteroalkyl-aryl group wherein heteroalkyl and aryl are as defined above, which may optionally be substituted with one or more substituents described as suitable substituents for each of the heteroalkyl and aryl.

"Cycloalkyl" means a non-aromatic mono- or multicyclic ring system comprising about 3 to about 20 carbon atoms, preferably about 3 to about 10 carbon atoms, particularly preferably about 5 to about 10 carbon atoms. Preferred cycloalkyl rings contain about 5 to about 7 ring atoms. The cycloalkyl can be optionally substituted with one or more "ring system substituents" which may be the same or different, and are as defined herein. Non-limiting examples of suitable monocyclic cycloalkyls include cyclopropyl, cyclopentyl, cyclohexyl, cycloheptyl and the like. Non-limiting examples of suitable multicyclic cycloalkyls include 1-decalinyl, norbornyl, adamantyl and the like, as well as partially saturated species such as, for example, indanyl, tetrahydronaphthyl and the like.

The term "heterocycloalkyl" as used herein, refers to a non-aromatic saturated monocyclic or multicyclic ring system comprising about 3 to about 20 carbon atoms, preferably about 3 to about 10 ring atoms, wherein from 1 to 4 of the ring atoms are independently O, S or N and the remainder of the ring atoms are carbon atoms. In one embodiment, a heterocycloalkyl group has from about 5 to about 10 ring atoms. In another embodiment, a heterocycloalkyl group has 5 or 6 ring atoms. There are no adjacent oxygen and/or sulfur atoms present in the ring system. Any -NH group in a heterocycloalkyl ring may exist protected such as, for example, as an -N(Boc), -N(CBz), -N(Tos) group and the like; such protected heterocycloalkyl groups are considered part of this invention. A heterocycloalkyl group can be optionally substituted by one or more "ring system substituents" which may be the same or different, and are as defined herein below. The nitrogen or sulfur atom of the heterocyclyl can be optionally oxidized to the corresponding N-oxide, S-oxide or S,S-dioxide. Examples of illustrative monocyclic heterocycloalkyl rings include piperidyl, pyrrolidinyl, piperazinyl, morpholinyl, thiomorpholinyl, thiazolidinyl, 1,4-dioxanyl, tetrahydrofuranyl, tetrahydrothiophenyl, lactam, lactone, and the like. A ring carbon atom of a heterocycloalkyl group may be functionalized as a carbonyl group.

The term "N-alkylamino" refers to a group comprising -C(O)-NH(alkyl) or -C(O)-N(alkyl)₂, such as

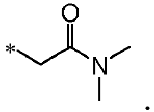

The term "inverted ester" refers to an ester moiety that is bound to the compound of Formula (I) or (II) via the O atom and preferably to a moiety selected from the group consisting of

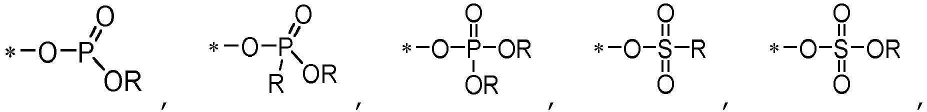

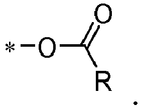

The term "substituted" means that one or more hydrogens on the designated atom is replaced with a selection from the indicated group, provided that the designated atom's normal valency under the existing circumstances is not exceeded, and that the substitution results in a stable compound. Combinations of substituents and/or variables are permissible only if such combinations result in stable compounds. By "stable compound' or "stable structure" a compound is meant that is sufficiently robust to survive isolation to a useful degree of purity from a reaction mixture, and formulation into an efficacious therapeutic agent.

"Halo" means fluorine, chlorine, bromine, or iodine. Preferred are fluorine, chlorine and bromine.

"Ring system substituent" means a substituent attached to an aromatic or non-aromatic ring system which, for example, replaces an available hydrogen on the ring system. Ring system substituents may be the same or different, each being independently selected from the group consisting of alkyl, alkenyl, alkynyl, aryl, heteroaryl, aralkyl, alkylaryl, heteroaralkyl, heteroarylalkenyl, heteroarylalkynyl, alkylheteroaryl, hydroxy, hydroxyalkyl, alkoxy, aryloxy, aralkoxy, acyl, aroyl, halo, nitro, cyano, carboxy, alkoxycarbonyl, aryloxycarbonyl, aralkoxycarbonyl, alkylsulfonyl, arylsulfonyl, heteroarylsulfonyl, alkylthio, arylthio, heteroarylthio, aralkylthio, heteroaralkylthio, cycloalkyl, heterocyclyl, -C(=N-CN)-NH₂, -C(=NH)-NH₂, -C(=NH)-NH(alkyl), Y₁Y₂N-, Y₁Y₂N-alkyl-, Y₁Y₂NC(O)-, Y₁Y₂NSO₂- and-SO₂NY₁Y₂, wherein Y₁ and Y₂ can be the same or different and are independently selected from the group consisting of hydrogen, alkyl, aryl, cycloalkyl, and aralkyl. "Ring system substituent" may also mean a single moiety which simultaneously replaces two available hydrogens on two adjacent carbon atoms (one H on each carbon) on a ring system. Examples of such moiety are methylene dioxy, ethylenedioxy, -C(CH₃)₂- and the like which form moieties such as, for example:

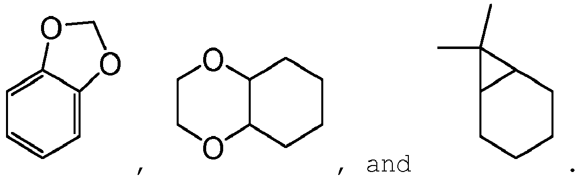

It should also be noted that any heteroatom with unsatisfied valences in the text, schemes and examples herein is assumed to have the hydrogen atom(s) to satisfy the valences.

When any variable (e.g., aryl, etc.) occurs more than one time in any constituent or in Formulas (I) to (IV), its definition on each occurrence is independent of its definition at every other occurrence.

Prodrugs and solvates of the compounds of the invention are also contemplated herein. The term "prodrug", as employed herein, denotes a compound that is a drug precursor which, upon administration to a subject, undergoes chemical conversion by metabolic or chemical processes to yield a compound of Formulas (I) to (IV) or a salt and/or solvate thereof.

"Solvate" means a physical association of a compound of this invention with one or more solvent molecules. This physical association involves varying degrees of ionic and covalent bonding, including hydrogen bonding. In certain instances the solvate will be capable of isolation, for example when one or more solvent molecules are incorporated in the crystal lattice of the crystalline solid. "Solvate" encompasses both solution-phase and isolatable solvates. Non-limiting examples of suitable solvates include ethanolates, methanolates, and the like. "Hydrate" is a solvate wherein the solvent molecule is H₂O.

The compounds of Formulas (I) to (IV) can form salts which are also within the scope of this invention. Reference to a compound of Formulas (I) to (IV) herein is understood to include reference to salts thereof, unless otherwise indicated. The term "salt(s)", as employed herein, denotes acidic salts formed with inorganic and/or organic acids, as well as basic salts formed with inorganic and/or organic bases. In addition, when a compound of Formulas (I) to (IV) contains both a basic moiety, such as, but not limited to a pyridine or imidazole, and an acidic moiety, such as, but not limited to a carboxylic acid, zwitterions ("inner salts") may be formed and are included within the term "salt(s)" as used herein. Pharmaceutically acceptable (i.e., non-toxic, physiologically acceptable) salts are preferred, although other salts can also be useful. Salts of the compounds of the Formulas (I) to (IV) may be formed, for example, by reacting a compound of the respective Formula with an amount of acid or base, such as an equivalent amount, in a medium such as one in which the salt precipitates or in an aqueous medium followed by lyophilization.

Exemplary acid addition salts include acetates, ascorbates, benzoates, benzenesulfonates, bisulfates, borates, butyrates, citrates, camphorates, camphorsulfonates, fumarates, hydrochlorides, hydrobromides, hydroiodides, lactates, maleates, methanesulfonates, naphthalenesulfonates, nitrates, oxalates, phosphates, propionates, salicylates, succinates, sulfates, tartarates, thiocyanates, toluenesulfonates (also known as tosylates,) and the like.

Exemplary basic salts include ammonium salts, alkali metal salts such as sodium, lithium, and potassium salts, alkaline earth metal salts such as calcium and magnesium salts, salts with organic bases (for example, organic amines) such as dicyclohexylamines, t-butyl amines, and salts with amino acids such as arginine, lysine and the like. Basic nitrogen-containing groups may be quarternized with agents such as lower alkyl halides (e.g. methyl, ethyl, and butyl chlorides, bromides and iodides), dialkyl sulfates (e.g. dimethyl, diethyl, and dibutyl sulfates), long chain halides (e.g. decyl, lauryl, and stearyl chlorides, bromides and iodides), aralkyl halides (e.g. benzyl and phenethyl bromides), and others.

All such acid salts and base salts are intended to be pharmaceutically acceptable salts within the scope of the invention and all acid and base salts are considered equivalent to the free forms of the corresponding compounds for purposes of the invention.

Compounds of Formulas (I) to (IV) and salts, solvates and prodrugs thereof, may exist in their tautomeric form (for example, as an amide or imino ether). All such tautomeric forms are contemplated herein as part of the present invention.

All stereoisomers (for example, geometric isomers, optical isomers and the like) of the present compounds (including those of the salts, solvates and prodrugs of the compounds as well as the salts and solvates of the prodrugs), such as those which may exist due to asymmetric carbons on various substituents, including enantiomeric forms (which may exist even in the absence of asymmetric carbons), rotameric forms, atropisomers, and diastereomeric forms, are contemplated within the scope of this invention, as are positional isomers (such as, for example, 4-pyridyl and 3-pyridyl). Individual stereoisomers of the compounds of the invention may, for example, be substantially free of other isomers, or may be admixed, for example, as racemates or with all other, or other selected, stereoisomers.

The pharmaceutical composition comprising the serine protease inhibitor may be administered in various forms. The serine protease inhibitor for use in preparing the pharmaceutical composition may be provided as micronized particles, wherein most particles preferably have a size of 50 µm or less, particularly 10 µm or less.

In a preferred embodiment of the present invention, the pharmaceutical composition comprises a serine protease inhibitor and one or more excipients from the group consisting of dissolving agents, mucoadhesives, hydrocolloids, lubricants, flow regulators, pH-adjusting agents, preservatives, dissolution control agents, flavoring agents, bulking agents, force control agents, gel-forming agents, solvents, thixotropic agents, stabilizers, osmotic agents, and particle-forming polymers.

In a preferred embodiment, the pharmaceutical composition is provided in the form of a lozenge. According to the present invention, a lozenge is a solid preparation that is intended to dissolve or disintegrate in the mouth. The lozenge may comprise a hard base material, e.g. including carbohydrates and/or polyols, or a soft base material, e.g. including arabic gum.

The lozenge preferably comprises 0.1 to 10 wt.-%, more preferably 0.2 to 5 wt.-%, such as 0.5 to 3 wt.-% of the serine protease inhibitor.

In a particularly preferred embodiment, the lozenge comprises one or more excipients selected from the group consisting of dissolving agents, mucoadhesives, hydrocolloids, lubricants, flow regulators, pH-adjusting agents, preservatives, and flavoring agents.

The lozenge preferably comprises one or more dissolving agents, i.e. excipients that can form a dissolving matrix suitable for being dissolved in the oral cavity, that typically comprise at least one of carbohydrates and/or polyols. Suitable dissolving agents may be selected from sucrose, glucose, lactose, fructose, maltose, trehalose, isomalt, maltitol, sorbitol, xylitol, mannitol, erythritol, and mixtures thereof. The lozenge preferably comprises 63 to 96.3 wt.-% of one or more dissolving agents.

Preferably, the lozenge further comprises a mucoadhesive, i.e. a component that can adhere to a mucosa, such as a hydrocolloid. Suitable mucoadhesives may be selected from xanthan, tragacanth, arabic gum, hyaluronic acid, sodium carboxymethyl cellulose, carmellose calcium (CMC) or carmellose sodium, hydroxypropylmethyl cellulose (HPMC), hydroxypropylcellulose, polyacrylic acid, polyvinylpyrrolidon (PVP), PVP/vinylacetat-copolymer, polyethylene glycol, chitosan, agarose, alginate, gelatin, and mixtures thereof. The lozenge preferably comprises 0 to 20 wt.-% of one or more mucoadhesives.

The lozenge may comprise a lubricant. Suitable lubricants may be selected from the group consisting of magnesium stearate, calcium behenate, zinc stearate, calcium stearate, talcum, and stearic acid. The lozenge preferably comprises 2 to 5 wt.-% of one or more lubricants. If the lozenge comprises polyethylene glycol as mucoadhesive, the polyethylene glycol may also have the effect of a lubricant.

The lozenge may further comprise a disintegrant. Suitable disintegrants may be selected from calcium hydogenphosphate, sodium hydrogencarbonate, sodium citrate, citric acid, and mixtures thereof. The lozenge preferably comprises 2 to 5 wt.-% of one or more disintegrants.

The lozenge may comprise a flow regulator. Suitable flow regulators may be selected from colloidal silica, talcum, and mixtures thereof. The lozenge preferably comprises 0.5 to 2 wt.-% of one or more flow regulators.

The lozenge may comprise a pH-adjusting agent. Suitable pH-adjusting agents may be selected from the group consisting of NaOH, citric acid, sodium hydrogenphosphate, glycin, trimethylglycine, and mixtures thereof. The lozenge preferably comprises 0.5 to 2 wt.-% of one or more pH-adjusting agents.

The lozenge may comprise a flavoring agent. Suitable flavoring agents may be selected from mint flavor, lemon flavor, cherry flavor and other commonly used flavoring agents. The lozenge preferably comprises 0.01 to 5 wt.-% of one or more flavoring agents.

The lozenge may comprise a preservative. Suitable preservatives may be selected from benzalkonium chloride and parabens.

According to a particularly preferred embodiment the lozenge comprises
(a) 0.2 to 5 wt.-% serine protease inhibitor, preferably in micronized form,
(b) 2 to 5 wt.-% lubricant, preferably calcium behenate,
(c) 0 to 20 wt.-% mucoadhesive, preferably HPMC and/or CMC,
(d) 1 to 5 wt.-% flavoring agent,
(e) 0.5 to 2 wt.-% flow regulator, preferably colloidal silica,
(f) 63 to 96.3 wt.-% dissolving agents, preferably sorbitol and/or mannitol.

The dissolving behavior of the lozenge typically depends on the type and amount of dissolution agent, disintegrant, lubricant and hydrocolloid used.

The taste of the lozenge typically depends on the used carbohydrates, polyols, and flavoring agents.

The lozenge may be prepared as a compressed lozenge, e.g. by direct compression or granulation-compression, or as a molten lozenge.

In another preferred embodiment, the pharmaceutical composition is provided in the form of a dry or liquid nasal preparation. The dry or liquid nasal preparation preferably comprises 0.01 to 90 wt.-% and more preferably 0.1 to 30 wt.-% or 0.1 to 20 wt.-% serine protease inhibitor. It is particularly preferred that a dry nasal preparation comprises 0.1 to 90 wt.-% serine protease inhibitor and that a liquid nasal composition comprises 5 to 20 wt.-% serine protease inhibitor.

In a preferred embodiment the pharmaceutical composition is a dry or liquid nasal composition comprising one or more excipients selected from the group consisting of bulking agents, mucoadhesives, force control agents, gel-forming agents, pH-adjusting agents, solvents, thixotropic agents, preservatives, flavoring agents, stabilizers, osmotic agents, and particle-forming polymers. Particularly preferably, the pharmaceutical composition is a dry nasal composition.

Preferably, the dry nasal preparation comprises a bulking agent, also referred to as filler, such as polyols and/or carbohydrates. Suitable bulking agents may be selected from mannitol, sorbitol, microcrystalline cellulose (MCC), lactose, colloidal MCC, trehalose, isomalt, maltitol, sucrose, glucose, dextran, maltodextrin, and mixtures thereof. The dry nasal composition preferably comprises 55 to 99 wt.-% of one or more bulking agents.

The dry or liquid nasal preparation may comprise a mucoadhesive, which may have the property of a gel-forming agent, a thixotropic agent and/or a particle-forming polymer. Suitable mucoadhesives may be selected from chitosan, pectin, polyacrylic acid (Carbomer), CMC, HPMC, HPC, agarose, alginate, hyaluronic acid, colloidal MCC, polyethylene glycol, polyvinylpyrrolidon (PVP), PVP/vinylacetat-copolymer, β-glucans, poly(lactic-co-glycolic acid), polycaprolactone, methacrylates, polyvinyl alcohol, and mixtures thereof. The dry nasal composition preferably comprises 0 to 50 wt.-% of one or more mucoadhesives.

In nasal preparations, mucoadhesives typically prolong the period after administration until a formulation is cleared from the nose towards the pharynx. Due to this nasal clearance mechanism, nasal preparations can typically also be effective in the pharynx and/or the larynx.

The dry nasal preparation may comprise a force control agent, i.e. an agents modifying the interfacial properties of the excipient particles to decrease drug-excipient adhesion. Suitable force control agents may be selected from magnesium stearate, calcium behenate, zinc stearate, calcium stearate, talcum, stearic acid, colloidal silica, micronized lactose and mixtures thereof. The dry nasal preparation preferably comprises 0 to 5 wt.-% of one or more force control agents.

The dry or liquid nasal preparation may comprise a pH-adjusting agent. Suitable pH-adjusting agents may be selected from NaOH, citric acid, sodium hydrogencarbonate, sodium hydrogenphosphate, glycin, trimethylglycine, and mixtures thereof. The liquid or dry nasal preparation preferably comprises 0 to 5 wt.-% of one or more pH-adjusting agents.

The liquid nasal preparation may further comprise a solvent. Suitable solvents may be selected from water, glycerol, ethanol, polyethylene glycol, soybean oil, and mixtures thereof. The liquid nasal preparation preferably comprises 70 to 95 wt.-% of one or more solvents. Dry nasal formulations may also comprise small amounts of solvents, which are typically residues from the preparation process of the components.

The liquid or dry nasal preparation may comprise an osmotic agent. Suitable osmotic agents may be selected from the group consisting of NaCl, sucrose, glycerol, and mixtures thereof. The liquid or dry nasal preparation preferably comprises 0 to 5 wt.-% of one or more osmotic agents.

The liquid or dry nasal preparation may comprise a preservative. Suitable preservatives may be selected from benzalkonium chloride, parabens, and mixtures thereof. The liquid or dry nasal preparation preferably comprises 0 to 1 wt.-% of one or more preservatives.

The liquid or dry nasal preparation may comprise a flavoring agent. Suitable flavoring agents may be selected from mint flavor, lemon flavor, cherry flavor, vanilla flavor, and other commonly used flavoring agents. The liquid or dry nasal preparation preferably comprises 0 to 5 wt.-% of one or more flavoring agents.

The liquid or dry nasal preparation may comprise a stabilizer. Suitable stabilizers are preferably selected from the group consisting of buffer substances, EDTA, polysorbate 80, polyvinyl alcohol, and mixtures thereof. The liquid or dry nasal preparation preferably comprises 0 to 5 wt.-% of one or more stabilizers.

According to a particularly preferred embodiment the dry nasal preparation comprises
(a) 0.1 to 10 wt.-% serine protease inhibitor that is preferably micronized,
(b) 0 to 5 wt.-% force control agent,
(c) 85 to 99.5 wt.-% bulking agent optionally comprising a mucoadhesive, preferably mannitol or MCC that has been mixed in equal parts by weight with pectin.

In another particularly preferred embodiment, the dry nasal preparation comprises the serine protease inhibitor, mannitol and a force control agent. Typically, this dry nasal preparation is easy to disperse and may be formed to a homogeneous blend.

In even another particularly preferred embodiment, the dry nasal preparation comprises the serine protease inhibitor, mannitol, a force control agent and charged mucoadhesive, such as chitosan, pectin, polyacrylic acid, CMC, and mixtures thereof, or an uncharged mucoadhesive, such as HPMC. Typically, this preparation exhibits increased mucoadhesivity and slower clearance compared to preparations without mucoadhesive.

According to another particularly preferred embodiment the liquid nasal preparation comprises
(a) 5 to 20 wt.-% serine protease inhibitor,
(b) 0.1 to 5 wt.-% mucoadhesive, preferably having thixotropic properties,
(c) 0 to 5 wt.-% osmotic agent and stabilizer combined,
(d) 70 to 94.9 wt.-% solvent or solvent mixture.

In another particularly preferred embodiment, the liquid nasal preparation comprises the serine protease inhibitor, water, buffer and benzalkonium chloride. Typically, this liquid nasal preparation allows for suitable concentrations and stability of serine protease inhibitors. Mucoadhesivity and residence time of the serine protease inhibitor on the mucosa may typically be increased by adding mucoadhesives, such as thixotropic agents and/or hydrophilic polymers.

Preferably, the pharmaceutical composition is a dry or liquid particulate nasal preparation comprising 2 to 50 wt.-% nano-or microspheres, which include the serine protease inhibitor. The liquid preparation, which includes nano- or microspheres, may comprise a solvent and one or more of a stabilizer, an osmotic agent, a buffer, and a preservative. The dry preparation, which includes nano- or microspheres, may comprise a mucoadhesive such as mannitol.

According to the present invention, nano- and microspheres are usually solid particles that comprise serine protease inhibitor and at least one excipient, preferably a particle-forming polymer, such as chitosan, alginate, carboxymethyl cellulose, poly(lactic-co-glycolic acid), methacrylates, or mixtures thereof, with nanospheres typically having sizes in the range of 1 to less than 1000 nm and microspheres typically having sizes in the range of 1 to 1000 µm.

In a preferred embodiment, the nano- or microspheres for use in a dry or liquid particulate nasal preparation comprise
(a) 2 to 50 wt.-% serine protease inhibitor,
(b) 0 to 5 wt.-% stabilizer,
(c) 45 to 98 wt.-% particle-forming polymers, such as chitosan, alginate, carboxymethyl cellulose, poly(lactic-co-glycolic acid), and methacrylates.

Nano- and microspheres may be prepared by ionic gelation, precipitation, the emulsion-diffusion method or spray drying.

The liquid and dry nasal preparations may be prepared and administered using any commonly used process or technology.

For example, dry nasal preparations may be prepared by low shear blending, high shear blending and sieving or by co-jet-milling.

Dry nasal preparations may be suitable for administration by use of suitable administration devices such as unit-dose devices (e.g. commercially available by Aptar Pharma) or a PowderJet^{®} device by Schuckmann, as described e.g. in WO 2004/033009 A1.

Liquid nasal preparations may be prepared using low-shear or high-shear liquid mixers, which may or may not have ultrasound functionality, such as an ULTRA-TURRAX^{®} homogenizer by IKA^{®}-Werke GmbH& Co. KG.

Liquid nasal preparations are preferably suitable for administration using a metered-dose nasal sprayer (e.g. commercially available by Ursapack, Aptar Pharma or Nemera) or using a unit-dose/bidose nasal sprayer (e.g. commercially available by Aptar Pharma).

In a second aspect, the invention relates to a serine protease inhibitor having a structure of Formula (III) 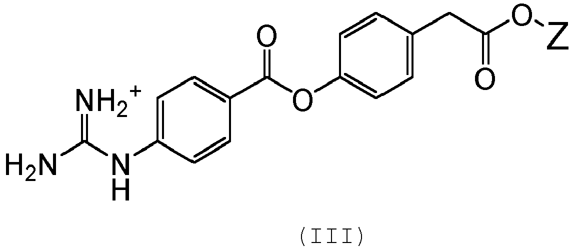 wherein Z is 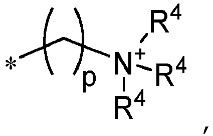
with p being an integer from 0 to 4, and
R⁴ being independently selected from the group consisting of H, alkyl, heteroalkyl, alkenyl, heteroalkenyl, alkynyl, heteroalkynyl, cycloalkyl, heterocycloalkyl, aryl, heteroaryl, alkylaryl, heteroalkylaryl, and preferably selected from H, alkyl and heteroalkyl, wherein said alkyl and heteroalkyl may have up to 3 carbon atoms, and more preferably R⁴ is methyl or H, and
more preferably Z is selected from the group consisting of 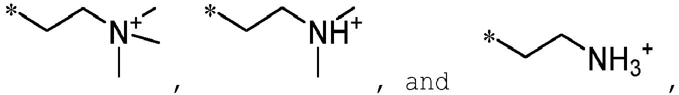 or a pharmaceutically acceptable salt thereof.

The compounds of the second aspect of the invention were found to have pharmacological properties. In particular, the compounds of Formula (III) are inhibitors of serine proteases. Hence, the compounds of Formula (III) are expected to be useful in the prevention or in the treatment of viral diseases, in particular in the prevention of a disease, disorder or condition caused by a virus affecting the upper respiratory tract, such as coronaviruses, influenza viruses, parainfluenza viruses, adenoviruses, rhinoviruses, respiratory syncytial viruses, in particular by coronaviruses and/or influenza viruses, as for instance by SARS-CoV-2, MERS-CoV and related coronaviruses, and particularly by SARS-CoV-2.

The serine protease inhibitors according to the second aspect of the invention may be obtained as follows:

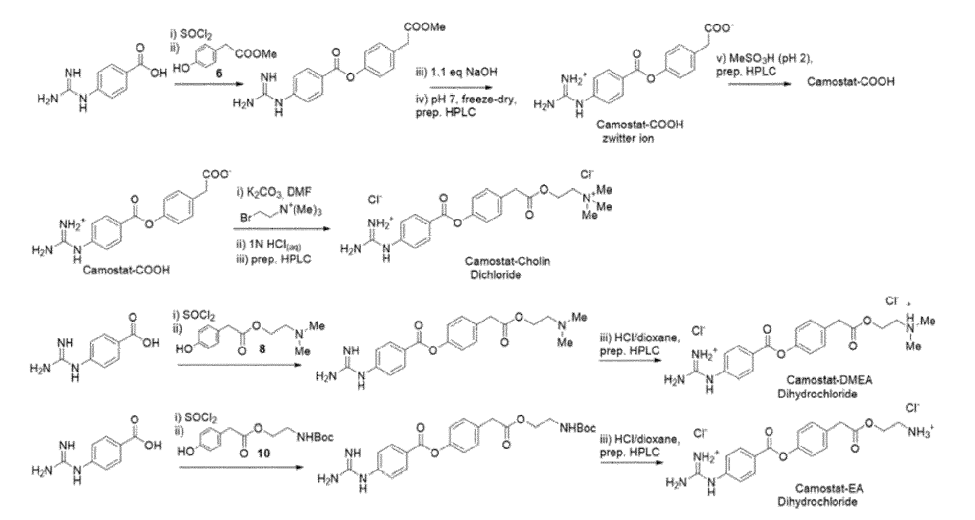

Analytical characterizations for identification of the compounds and assessing the purity may be performed by standard methods, such as LC/MS and NMR. For instance, samples may be analyzed using an Interchim puri-FLASH 4250/prep-HPLC system, a 400 MHz NMR (e.g. by Bruker) or an Amazon SL MS (Bruker).

In a third aspect, the present invention is directed to a lozenge comprising a serine protease inhibitor according to the second aspect of the invention, preferably in an amount of 20 mg or less, particularly 0.05 to 10 mg, and particularly preferably 0.2 to 5 mg.

All compositions, material definitions, and processes for preparation, in each case including all preferred embodiments, which were described for the pharmaceutical composition in the form of a lozenge according to the second aspect of the invention, are also suitable for the lozenge according to the third aspect of the invention.

Furthermore, the present invention is in a fourth aspect directed to a dry or liquid nasal preparation comprising a serine protease inhibitor according to the second aspect of the invention, preferably in an amount of 1mg µg or less, particularly 1 to 500 µg, and particularly preferably 5 to 200 µg per single dosage form. It is preferred that the nasal preparation is a dry nasal preparation.

All compositions, material definitions, and processes for preparation, in each case including all preferred embodiments, which were described for the pharmaceutical composition in the form of a dry nasal preparation according to the second aspect of the invention, are also suitable for the dry nasal preparation according to the fourth aspect of the invention.

The invention disclosed herein is exemplified by the following examples which should not be construed to limit the scope of the disclosure. Alternative mechanistic pathways and analogous structures will be apparent to those skilled in the art.

### Examples

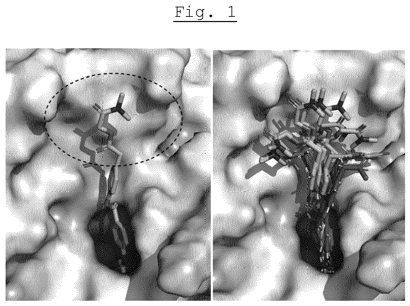

Camostat and the compound having the structure of 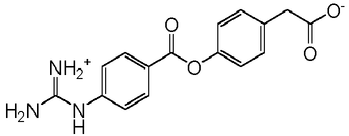 , which is a metabolite of camostat, were docked into the active site of a serine protease TMPRSS2, whose structure was created on the basis of a homology model, using the Rosetta software suite. The left panel of Fig. 1 shows camostat docked into the active site of TMPRSS2.

The right panel of Fig. 1 is an overlay of camostat mesylate and the aforementioned metabolite docked into the active site of TMPRSS2. It can be seen that the distal amide of the serine protease inhibitors, such as the distal dimethylamide moiety of camostat, does not considerably contribute to binding. According to these data, especially the distal amide group of the serine protease inhibitors can be exchanged with various chemical groups without considerably affecting the binding to the target protein and thus the inhibitory activity.

## Claims

1. Pharmaceutical composition comprising a serine protease inhibitor for use in a method for the treatment or prevention of viral infections, wherein the pharmaceutical composition is administered topically.

2. Pharmaceutical composition according to claim 1 that is topically administered to a mucosa, preferably to a mucosa of the upper respiratory tract or the eye, particularly preferably to a mucosa of the upper respiratory tract.

3. Pharmaceutical composition according to claim 1 or 2, wherein the viral infection is caused by a virus infecting the respiratory tract, in particular a virus selected from the group consisting of coronaviruses, influenza viruses, parainfluenza viruses, adenoviruses, rhinoviruses, respiratory syncytial viruses, more preferably by coronaviruses and/or influenza viruses, particularly preferably by SARS-CoV-2, MERS-CoV and related coronaviruses, and most preferably by SARS-CoV-2.

4. Pharmaceutical composition according to any one of claims 1 to 3 that is administered in the form of a lozenge, a mucoadhesive buccal preparation, a medicated chewing gum, a nasal preparation, an inhalation product, an ophthalmic ointment or eye drops, preferably as a lozenge or as a nasal preparation,
wherein the pharmaceutical composition is preferably administered in the form of a lozenge with a daily maximum dose of serine protease inhibitor of 300 mg or less, more preferably 250 mg or less, and particularly preferably 200 mg or less, or in the form of a nasal preparation with a maximum daily dose of 100 mg or less, more preferably 30 mg or less, and particularly preferably 1 mg or less, and/or
wherein the pharmaceutical composition is preferably administered in the form of a lozenge having an absolute dose of serine protease inhibitor per single dosage form of 20 mg or less, more preferably 0.05 to 10 mg, and particularly preferably 0.2 to 5 mg, or in the form of a nasal preparation having an absolute dose of serine protease inhibitor per single dosage form of 1 mg or less, more preferably 1 to 500 µg, and particularly preferably 5 to 200 µg.

5. Pharmaceutical composition according to any one of claims 1 to 6, wherein the administration of a single dosage form of the pharmaceutical composition to a fasted individual provides a peak plasma concentration (Cₘₐₓ) of the serine protease inhibitor and its active metabolites combined of 0.3 µM or less, preferably 0.1 µM or less, particularly preferably 0.03 µM or less, and most preferably 0.01 µM or less.

6. Pharmaceutical composition according to any one of claims 1 to 7, wherein the serine protease inhibited by the serine protease inhibitor is a transmembrane protease, preferably a type II transmembrane protease, TMPRSS2.

7. Pharmaceutical composition according to any one of claims 1 to 6, wherein the serine protease inhibitor is a compound of Formula (I) or (II) 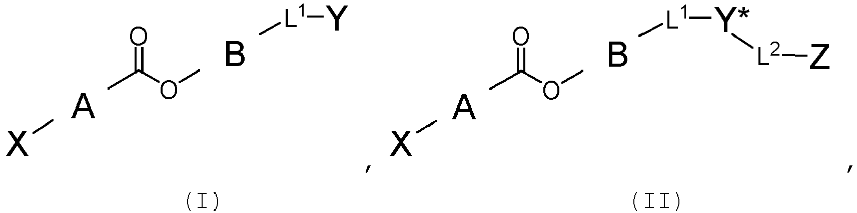
wherein A and B are independently selected from aryl and heteroaryl,
X is selected from the group consisting of 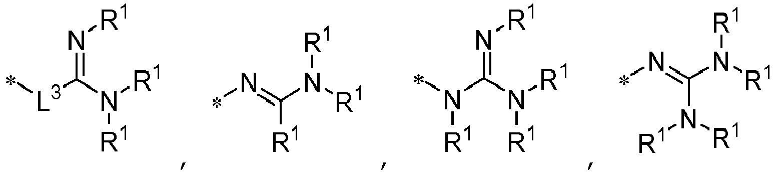 and 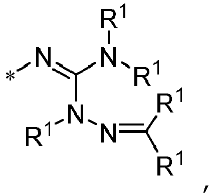 wherein
L³ is an optional linking group selected from the group consisting of alkyl, heteroalkyl, alkenyl, heteroalkenyl, alkynyl, alkylaryl, heteroalkylaryl, heteroalkynyl, cycloalkyl, heterocycloalkyl, aryl, and heteroaryl, and preferably is a linear or branched alkyl having up to 4 carbon atoms, more preferably 1 or 2 carbon atoms, and
R¹ is independently selected from the group consisting of H, alkyl, heteroalkyl, alkenyl, heteroalkenyl, alkynyl, heteroalkynyl, cycloalkyl, heterocycloalkyl, aryl, heteroaryl, alkylaryl, heteroalkylaryl, and preferably selected from H, alkyl and heteroalkyl, wherein said alkyl and heteroalkyl have up to 3 carbon atoms and are optionally joined to another R¹ to form a heterocycle, and more preferably R¹ is H,
Y is an acidic functional group, preferably selected from the group consisting of carboxylic acid, sulfonic acid, phosphonic acid, phosphinic acid, sulfonamides, and hydroxamic acid, and esters and inverted esters thereof,
Y* is a hydrolysable derivative from an acidic functional group, preferably selected from the group consisting of ester, inverted ester, amide, carbamate, and anhydride,
Z is an N-containing group and is preferably selected from the group consisting of N-containing heteroalkyl and alkylamide moieties, each having preferably up to 6 carbon atoms, and N-containing monocyclic and bicyclic heterocyclyl and heteroaryl, and
L¹ and L² are optional linking groups which are present or absent and which are independently selected from the group consisting of alkyl, heteroalkyl, alkenyl, heteroalkenyl, alkynyl, alkylaryl, heteroalkylaryl, heteroalkynyl, cycloalkyl, heterocycloalkyl, aryl, and heteroaryl, and preferably are linear or branched alkyl having up to 4 carbon atoms,
or a pharmaceutically acceptable salt thereof.

8. Pharmaceutical composition according to claim 7, wherein the serine protease inhibitor is a compound of Formula (I) or (II), wherein
A and B are independently selected from monocyclic, bicyclic or tricyclic aryl or heteroaryl and are preferably phenyl or naphthyl, particularly preferably phenyl,
Y is selected from the group consisting of 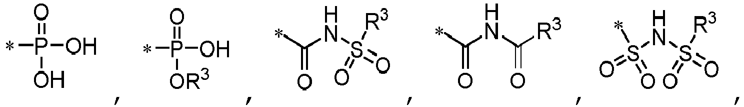 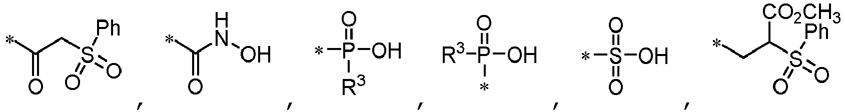 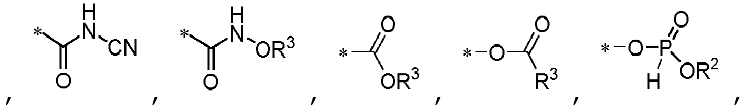 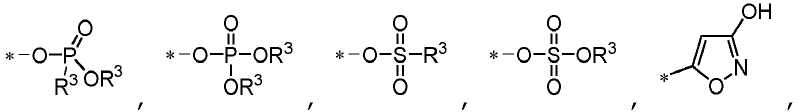 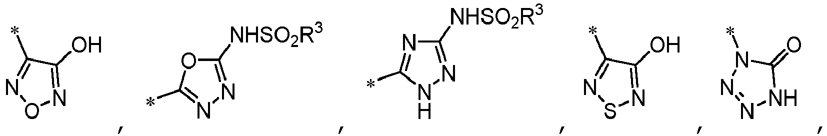 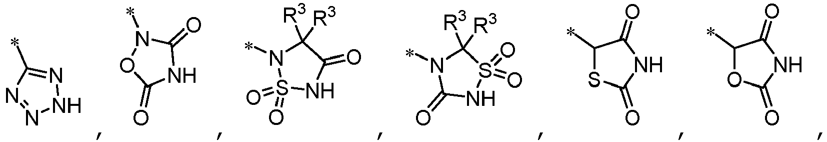 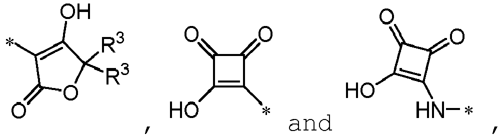 R³ is independently selected from the group consisting of H, alkyl, heteroalkyl, alkenyl, heteroalkenyl, alkynyl, heteroalkynyl, cycloalkyl, heterocycloalkyl, aryl, heteroaryl, alkylaryl, heteroalkylaryl, and preferably selected from the group consisting of H, alkyl, heteroalkyl, cycloalkyl and heterocycloalkyl, wherein said alkyl, heteroalkyl, cycloalkyl or heterocycloalkyl can have up to 6 carbon atoms,
Y* is an ester group or an inverted ester group, preferably a carboxylic acid ester, sulphonic acid ester, phosphonic acid ester, phosphoric acid ester or phosphonic acid diester, and more preferably a carboxylic acid ester,
Z is selected from N-alkylamino and quaternary ammonium moieties and preferably from 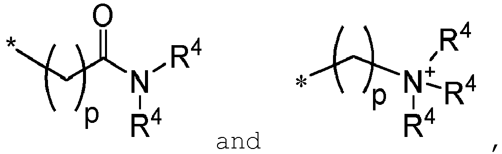
wherein p is an integer from 0 to 4, preferably 1 or 2, and
R⁴ is independently selected from the group consisting of H, alkyl, heteroalkyl, alkenyl, heteroalkenyl, alkynyl, heteroalkynyl, cycloalkyl, heterocycloalkyl, aryl, heteroaryl, alkylaryl, heteroalkylaryl, and preferably selected from H, alkyl and heteroalkyl, wherein said alkyl and heteroalkyl may have up to 3 carbon atoms, and is optionally joined to L² or to another R⁴ to form a heterocycle, and more preferably R⁴ is methyl or H, and
more preferably Z is selected from the group consisting of 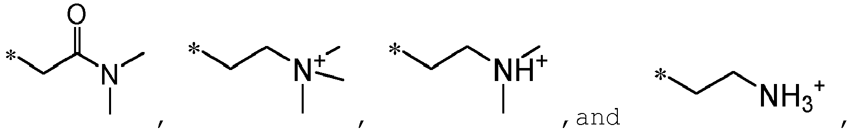 with L² being absent,
or a pharmaceutically acceptable salt thereof.

9. Pharmaceutical composition according to claim 8, wherein the serine protease inhibitor is a compound having a structure of Formula (III) 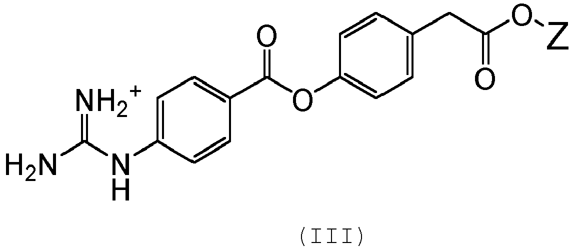
wherein Z is 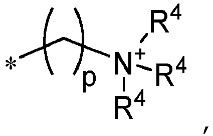
with p being an integer from 0 to 4, and
R⁴ being independently selected from the group consisting of H, alkyl, heteroalkyl, alkenyl, heteroalkenyl, alkynyl, heteroalkynyl, cycloalkyl, heterocycloalkyl, aryl, heteroaryl, alkylaryl, heteroalkylaryl, and preferably selected from H, alkyl and heteroalkyl, wherein said alkyl and heteroalkyl may have up to 3 carbon atoms, and more preferably R⁴ is methyl or H, and
more preferably Z is selected from the group consisting of 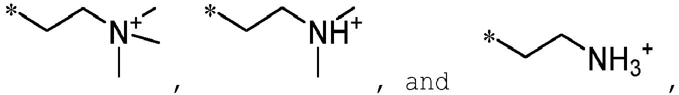 or a pharmaceutically acceptable salt thereof.

10. Pharmaceutical composition according to any one of claims 1 to 9, wherein the serine protease inhibitor is present as a zwitterion at a pH value that is in the range of pH 5.5 to pH 7.5.

11. Pharmaceutical composition according to any one of claims 1 to 10, wherein the pharmaceutical composition is a lozenge comprising at least one excipient selected from the group consisting of dissolving agent, mucoadhesives, hydrocolloids, lubricants, flow regulators, pH-adjusting agents, preservatives, and flavoring agents.

12. Pharmaceutical composition according to any one of claims 1 to 10, wherein the pharmaceutical composition is a dry or liquid nasal preparation comprising at least one excipient from the group consisting of bulking agents, mucoadhesives, force control agents, gel-forming agents, pH-adjusting agents, solvents, thixotropic agents, preservatives, flavoring agents, stabilizers, osmotic agents, and particle-forming polymers.

13. Serine protease inhibitor that is a compound having a structure of Formula (III) 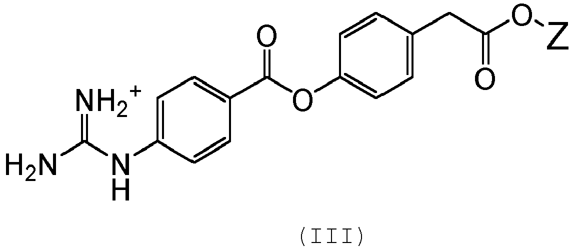
wherein Z is 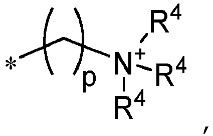
with p being an integer from 0 to 4, and
R⁴ being independently selected from the group consisting of H, alkyl, heteroalkyl, alkenyl, heteroalkenyl, alkynyl, heteroalkynyl, cycloalkyl, heterocycloalkyl, aryl, heteroaryl, alkylaryl, heteroalkylaryl, and preferably selected from H, alkyl and heteroalkyl, wherein said alkyl and heteroalkyl may have up to 3 carbon atoms, and more preferably R⁴ is methyl or H, and
more preferably Z is selected from the group consisting of 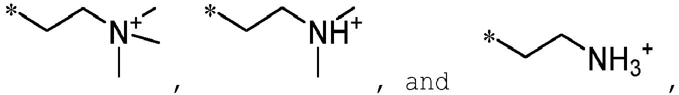 or a pharmaceutically acceptable salt thereof.

14. Lozenge comprising a serine protease inhibitor according to claim 13, preferably in an amount of 20 mg or less, particularly 0.05 to 10 mg, and particularly preferably 0.2 to 5 mg.

15. Dry or liquid nasal preparation comprising a serine protease inhibitor according to claim 13, preferably in an amount of 1 mg or less, particularly 1 to 500 µg, and particularly preferably 5 to 200 µg per single dosage form.
